# EUROPEAN PATENT SPECIFICATION

(11) **EP 3 570 801 B1**
(45) Date of publication and mention of the grant of the patent: **04.01.2023**
(21) Application number: 17892486.6
(22) Date of filing: 20.01.2017
(51) Int. Cl.: A61F 13/64, A61F 13/49, A61F 13/58

(54) **DISPOSABLE ARTICLE**
EINWEGARTIKEL
ARTICLE JETABLE

(43) Date of publication of application: 27.11.2019
(73) Proprietor: ESSITY HYGIENE AND HEALTH AKTIEBOLAG, 405 03 Göteborg (SE)
(72) Inventor: MEYER NORÉN, Rikard Vilhelm, 411 01 Göteborg (SE); DOUGHERTY, Eugene, Philadelphia, PA 19104 (US)
(74) Representative: Nederlandsch Octrooibureau
(86) International application number: PCT/SE2017/050058
(87) International publication number: WO 2018/135984

(56) References cited:
- EP-A2- 1 543 804
- WO-A1-99/37263
- WO-A1-2007/071267
- WO-A1-2010/071507
- US-A1- 2008 208 152
- US-A1- 2012 172 828
- US-A1- 2015 202 091

## Description

### TECHNICAL FIELD

This disclosure relates to a disposable absorbent article comprising a chassis having first and second end portions and a central portion extending therebetween. The article further comprises a first side panel and a second side panel extending from opposite sides of the first end portion of the chassis. The first and second side panels are folded over the chassis to form a folded side panel configuration prior to use of the article. The first side panel is arranged at least partly on top of the second side panel in the folded side panel configuration.

### BACKGROUND

Absorbent products comprising side panels are today quite common on so-called open absorbent articles and known to those skilled in the art. By using side panels instead of fastening tabs it is possible to make the chassis of the product narrower which saves both material cost as well as makes the product more pleasant to wear since the product does not cover as much of the users' skin. Another advantage is the fact that side panels can easily be tailored to have desired characteristics such as elasticity or breathability.

One drawback with side panels relates to the production problem of absorbent products. Absorbent products are produced at very high speeds and side panels extending outwards in a lateral direction can cause problems such as jamming of the machine with the material pieces. This problem can be overcome by releasably bonding the loose distal edges of the side panels in the process, the bonds at a later stage being released by the user when putting on the article. The releasable bond/attachment may sometimes be referred to as a breakable bond. Such breakable bonds are known for example from WO2010/071507.

In view of this document there is still a need for a product which has side panels releasably bonded during manufacturing and transportation, but that offers easy release of the bonds once the product is to be used. At the same time the manufacturing of articles with such side panels should be easy without including too many complex processing steps.

### SUMMARY

An object of the present disclosure is to provide an improved disposable article, in which the side panels are easy and quick to open up when the side panels are in a folded side panel configuration.

The disclosure concerns a disposable article. The disposable article comprises a chassis having first and second end portions and a central portion extending therebetween, the article further comprising a first side panel and a second side panel extending from opposite sides of the first end portion of the chassis, the first and second side panels being folded over the chassis to form a folded side panel configuration prior to use of the article. Moreover, the first side panel is arranged at least partly on top of the second side panel to form an overlap area in the folded side panel configuration, wherein the second side panel is bonded to the chassis by a first breakable bond in the folded side panel configuration and the first side panel is bonded to the second side panel by a second breakable bond in the overlap area. The first breakable bond has a weaker peel strength than the second breakable bond.

In other words, the second side panel is folded over the chassis and the first side panel is folded over the second side panel such that at least a part of the first and second side panels or the entire first and second side panels overlap. The first side panel may be arranged partly on top of the second side panel or entirely on top of the second side panel.

The first and second side panels, sometimes referred to as the first pair of side panels, may be attached to the chassis. Alternatively, the first and second side panels may be integral parts of the chassis extending on each side of the first end portion of the chassis. In this manner, a wearer or caregiver is provided with a disposable article which is easy to get into a "ready-to-use" non-folded side panel configuration. In other words, the side panels are easily released and unfolded from the folded side panel configuration. Thus, by the provision of having the first breakable bond being of weaker peel strength than the second breakable bond both breakable bonds will break as the user pulls a free distal edge of the first side panel which is the only distal edge available to the user when the article is in the folded side panel configuration. The benefit of releasing both breakable bonds with one simple movement is achieved. This is in contrast to an embodiment in which the peel strengths of the first and second breakable bonds are the same. In such an embodiment it is likely that the second breakable bond between the first and second side panel will break before the first breakable bond between the second side panel and the chassis. In such a case, the release of the bonds must be performed in two steps; grabbing and pulling the first side panel and subsequently grabbing and pulling the first side panel.

To this end, the exemplary embodiments of the disclosure provide the advantage that less consideration is needed to find and grab the distal edge of the second side panel once the distal edge of the first side panel has been found and pulled when the article is in the folded side panel configuration.

Accordingly, the exemplary embodiments enable a user to easily transform the article from the folded side panel configuration into the ready-to-use state. Thus, preparing the disposable article for use may be done in a more straight forward and intuitive manner requiring less time and effort of the wearer or caregiver.

Further advantages are achieved by implementing one or several of the features of the dependent claims.

The terms "breakable bonds" and "releasably bonded" as used herein are regarded as interchangeable and indicates a bond between two surfaces or parts, or a combination thereof that, on the one hand, can be easily broken by a user by simply pulling apart one side panel from the other but, on the other hand, is strong enough to withstand any forces acting on the side panels during the manufacturing process. Such a bond can be formed, for example, by a breakable adhesive, spot welding, thermobonding, compression bonding, ultra-sonic welding or the like, and typically leads to a bond that cannot be refastened once it has been broken. It is to be understood that a breakable bond is a temporary bond, i.e. the side panels are temporarily bonded.

It is to be understood that the exemplary embodiments of the disclosure may be implemented in an open diaper to be closed by means of a fastening means such as the side panels or a belt. The exemplary embodiments may further be implemented in incontinence guards.

In some exemplary embodiments, the first side panel and the second side panel are fastened or secured or pegged together with at least one breakable bond in the overlap when the side panels are in the folded side panel configuration. This releasable attachment in the overlap ensures that the free end portions of the side panels do not move around before they are unfolded. Any potentially dangling loose ends of the free end portions of the side panels are typically appropriately fastened such that the free ends are confined to one specific location or area within the boundaries of chassis of the article.

The article has a body-oriented surface facing the user during use and a garment-oriented surface facing away from the user in use.

In one variant, the second side panel is folded over the body-oriented surface when the article is in the folded side panel configuration. The second side panel is releasably bonded to the body-oriented surface by a breakable bond.

In one variant, the second side panel is folded over the garment-oriented surface when the article is in the folded side panel configuration. The second side panel is releasably bonded to the garment-oriented surface by a breakable bond.

As mentioned above, the term "folded side panel configuration" of the first and second side panels refers to an orientation of the side panels prior to use of the article. Typically, although not strictly required, the folded side panel configuration of the side panels is formed during manufacturing and assembling of the article. The side panels of the article can be folded in several different manners in order to form the side panel configuration, as further described herein.

Typically, the folded side panel configuration may be formed by the at least one side panel being folded over the body-oriented surface. Alternatively the folded side panel configuration may be formed by the at least one side panel being folded or over the garment-oriented surface. In other words, when the side panels are in the folded side panel configuration prior to use, the at least one side panel is folded over the body-oriented surface or over the garment-oriented surface. Analogously, when the side panels are in the folded side panel configuration prior to use, the other one of the side panel is folded over the side panel. The product may comprise two or four side panels, and each pair may be folded into a folded side panel configuration as described herein.

The first and second side panels comprise distal edges, respectively. Each one of the distal edges of the first and second side panels being folded over and beyond the longitudinal central line when the article is in the folded side panel configuration prior to use such that the overlap area is centrally arranged along the longitudinal central line of the disposable article.

In one example the first breakable bond is arranged in the overlap area when the article is in the folded side panel configuration. Thus the first and second side panels may be securely attached to the absorbent article during production. Alternatively the first breakable bond may be arranged in an area, outside the overlap area. However, such a placement of the first breakable bond, being outside the overlap area may result in that a portion of the first and second panels comes loose from or in another way moves away from the chassis of the absorbent article during production which increases the risk for the first and second side panels to interfere with manufacturing equipment further down in the production line. Such intereference may cause the need for production to be stopped while the interfering article is being removed, thereby increasing down time of the production, which in turn results in increased manufacturing costs. Further, the inventors have realized that placing the first breakable bond in the overlap area may allow for the first and second panels to be removed by the wearer or caregiver in a simpler and more intuitive manner.

In one example, the second breakable bond is arranged on top of the first breakable bond when the article is in the folded side panel configuration prior to use. Such a configuration simplifies the manufacturing of the article with respect to the breakable bonds. Moreover, the first breakable bond, having weaker peel strength than the second breakable bond, will easily break before the second breakable bond if they are arranged on top of each other. In other words, the second side panel will easily detach from the chassis at the first breakable bond but remain bonded to the first side panel by means of the second breakable bond when a user attempts to pull the distal edge of the second side panel available to be pulled when the article is in the folded side panel configuration.

In one example, the breakable bonds are arranged adjacent or on the longitudinal central line of the disposable article. In the example the breakable bonds are closer to a longitudinal central line than they are close to the fold line of the side panel. Typically, the breakable bonds are arranged within a distance of 30% of the full folded side panel length, from the longitudinal central line. A distance of 100% is defined by the distance from the fold line of the first side panel or second side panel to the longitudinal central line.

In one exemplary embodiment, the first breakable bond is arranged in a pattern that covers the entire second side panel in the overlap area. That is, the breakable bond pattern covers the part of the second side panel facing the chassis and that is arranged below the first side panel being folded on top of it. In another example, the bonding pattern of the first breakable bond covers the entire second side panel.

In one exemplary embodiment the second breakable bond is arranged in a pattern that covers the entire first side panel in the overlap area. That is, the breakable bond pattern covers the part of the first side panel facing the second side panel and that is arranged on top of the second side panel in an overlap configuration.

In one exemplary embodiment, a force of 2.5-8 N is required to rupture the first breakable bonds and a force 7-22 N is required to rupture the second breakable bonds. Preferably, a force of 3-7 N is required to rupture the first breaking bonds and a force 8-18 N is required to rupture the second breaking bonds. Most preferably, a force of 4-6 N is required to rupture the first breakable bonds and a force 7-14 N is required to rupture the second breaking bonds. Alternatively, the forces required to rupture the first and second breakakable bonds is selected in the range between 2.5-22 N, wherein the force required to rupture the first breakable bond is higher than the force required for ruptureing the second breakable bond. The second breakable bond is stronger than the first breakable bond, thereby ensuring that the first breakable bond breaks and detaches the second side panel from the chassis before the second breakable bond breaks and detaches the first side panel form the second side panel. Thereby the first and the second bonds can be broken sequentially by one single pulling motion which facilitates the release of the side panels and allows the article to be ready for use.

Additionally, the force required for rupturing the first breakable bond may be defined as a factor of the force required for rupturing the second breakable bond. For instance, the force required for rupturing the first breakable bond may be between 10-90% of the force required for rupturing the second breakable bond. Additionally, the force required for rupturing the first breakable bond may be between 20-80% of the force require for rupturing the second breakable bond, preferably the force required for rupturing the first breakable bond may be between 20-70% of the force require for rupturing the second breakable bond, more preferably the force required for rupturing the first breakable bond may be between 30-60% of the force require for rupturing the second breakable bond.

In one exemplary embodiment the first breakable bond is an integral part of the second side panel. In one exemplary embodiment the second breakable bond is an integral part of the first side panel. In one exemplary embodiment the first breakable bond is an integral part of the second side panel and the second breakable bond is an integral part of the first side panel. This is achievable by for example welding such as ultrasound welding, thermobonding or compression bonding. Having the first and/or the second breakable bond in the form of an integral part of the first and/or second side panel reduces the need to add a separate bonding material. This, in turn may reduce the manufacturing cost for producing the absorbent article.

In one exemplary embodiment the first breakable bond is a separate part of the second side panel. In one exemplary embodiment the second breakable bond is a separate part of the first side panel. In one exemplary embodiment the first breakable bond is a separate part of the second side panel and the second breakable bond is a separate part of the first side panel. This is achievable by, for example, adhesives.

The first and second breakable bonds may be a combination of both integral parts of the side panels as well as separate parts of the side panels. Moreover, the first breakable bond may be an integral part of the second side panel and the second breakable bond may be a separate part of the first side panel, or vice versa.

Both side panels may be folded in an accordion-like fashion, for example folded in a Z-shape, M-shape, W-shape or the like, over the body-oriented surface or over the garment-oriented surface. The side panels may be folded in in the same fold, or in different folds.

In some exemplary embodiments, at least one of the first and second side panels is folded in order to wrap up a length of the at least one side panel. Typically, the at least one side panel is folded in an accordion-like fashion in order to wrap up a length of the side panel. The accordion-like fold can be formed in several different manners, for example being folded in a Z-shape, M-shape, W-shape or the like, over the body-oriented surface or over the garment-oriented surface. Typically, accordion folds refer to zig-zag folds with at least two parallel folds that go in opposite directions. Although the length of each folded panel may be about the same size, it should be readily appreciated that the term accordion fold also includes variants in which the folded panels are of different sizes, a higher number of parallel folds, and/or a combination thereof. Further, the folded panels making up the accordion fold may be held together by the releasable attachment, as mentioned above. By the above wrap up configuration of a side panel, it becomes possible to accommodate side panel lengths. Folding the side panels, especially if they form belt portions, ensures that even longer side panels can be confined within the boundaries of the chassis surface. In this context, it may be particularly advantageous to fold the side panel in a Z-shape such that the front end of the free end portion is situated in the direction of the longitudinal extension of the side panel such that a user can easily grasp the free end portion. This arrangement is also more intuitive for a user since the free end portion of the side panel extends in the direction in which the user expects it to unfold.

The first and second side panels may also be folded in other ways when forming the folded side panel configuration prior to use of the article. For instance, each one of the first side panel and the second side panel may be double folded. Alternatively, the second side panel may be double folded over the chassis, whilst the first side panel is folded over the second side panel. This is in particular beneficial if the second side panel is longer than the first side panel which might be the case for example if the side panels function as belt portions.

In one exemplary embodiment, the longitudinal extension of the side panel is equal to, or is longer than the maximum lateral extension of the chassis. The arrangement of the side panels as described above is particularly advantageous with reasonably long side panels, in particular belt portions of the length being equal to, or longer, than the maximum lateral extension of the chassis because any clogging or jamming of the production and/or packaging lines can be prevented. Longer belt portions can be more comfortable to wear for a wearer under certain circumstances.

Any one of the first and second side panels may comprise a corresponding intermediate elastic region. The side panels can, in accordance with other alternative aspects of the disclosure, be inelastic or partly elastic. A partly elastic side panel means that certain parts of the length of the side panel have elastic properties, while certain other parts of the length of the side panel do not have elastic properties. In some design variants, any one of the first and second side panels comprises an intermediate elastic region.

In one exemplary embodiment the disposable article is a disposable absorbent article such as a diaper; the diaper being an open-diaper or belted-type diaper, and an incontinence guard. The article may be intended for a young child or a baby or adults.

A side panel-opening trigger zone may be arranged on the exterior surface of the first side panel. The side panel-opening trigger zone is disposed adjacent a free end of the first side panel. The side panel-opening trigger zone indicates where the first side panel is intended to be pulled in order to release the breakable bonds in when the article is in the folded side panel configuration prior to use.

In some design variants, a part of the free end adjacent a free end edge of the first side panel is distinguished from the remaining parts of the article by colour, pattern or the like.

Typically, although not strictly necessary, a part of any one of the first and second side panels, which extends from the first end portion of the article, is permanently attached to the first end portion.

In some exemplary embodiments, the article has an absorption body arranged between a first liquid-permeable covering layer and a second substantially liquid-impermeable covering layer. Typically, the first liquid-permeable covering layer forms the body-oriented surface, whilst the second substantially liquid-impermeable covering layer forms the garment-oriented surface.

In one exemplary embodiment the article comprises a second pair of side panels attached to the chassis so that a third side panel and a fourth side panel extend on each side of the second end portion of the chassis. The second pair of side panels may be provided with breakable bonds accordingly with the first pair of side panels, as disclosed herein. That means that a third and a forth side panel are folded over the chassis, to form a folded side panel configuration prior to use of the article, wherein the fourth side panel is folded over the chassis and the third side panel is arranged at least partly or entirely on top of the forth side panel to form an overlap area in the folded side panel configuration.

The side panels of the first pair of side panels may carry a respective first and second fastener adapted to be attached to the side panels of the second pair of side panels so that the article assumes a pant-like shape when in use. Any one of the side panels may carry a fastener adapted to be attached to an exterior surface of another side panel.

In some exemplary embodiments, the first and second fasteners are arranged on the distal edge of the respective first and second side panel, and the distal edge of the first side panel is folded over the first fastener such as to cover the first fastener when the article is in the folded side panel configuration, and the distal edge of the second side panel is folded over the second fastener such as to cover the second fastener when the article is in the folded side panel configuration. In some exemplary embodiments, the first breakable bonds are arranged on the part of the first side panel that covers the first fastener and the second breakable bonds are arranged on the part of the second side panel that covers the second fastener.

In one exemplary embodiment, the side panels of the first pair of side panels carry a fastener adapted to be attached to an exterior surface of the other of the side panels of the first pair of side panels, and wherein the second end portion of the chassis comprises a fastening device for securing the second end portion of the chassis to one of the side panels so that the article assumes a pant-like shape with the side panels forming a part of a waist portion of the pant when in use. The side panels may form long belt portions. Separate belt portions may also be attached to the side panels such as to elongate the side panels into belt portions.

In one exemplary embodiment, the first and second side panels form belt portions of a belted article.

Further features of, and advantages with, the exemplary embodiments of the present disclosure will become apparent when studying the appended claims and the following description. The skilled person will realize that different features of the exemplary embodiments may be combined to create embodiments other than those described in the following, without departing from the scope of the present disclosure.

### BRIEF DESCRIPTION OF THE DRAWINGS

With reference to the appended drawings, below follows a more detailed description of embodiments of the disclosure cited as examples.

In the drawings:
Fig. 1 schematically shows an exemplary embodiment of a disposable article of a belt type comprising a first side panel and a second side panel according to the disclosure from the side that is intended to face towards the user when in use;
Fig. 2 shows an exemplary embodiment of a disposable article of an open-diaper type comprising side panels according to the disclosure from the side that is intended to face towards the user when in use, and in which a first side panel and a second side panel is folded over the chassis to form a folded side panel configuration;
Fig. 3A schematically shows an exploded view of the first side panel and the second side panel according to an exemplary embodiment of the disclosure;
Fig. 3B schematically shows an exploded view of the first side panel and the second side panel according to another exemplary embodiment of the disclosure;
Figs. 4-5 schematically show various exemplary embodiments of a disposable article comprising side panels according to the disclosure, in which the side panels are folded in several different ways to form the folded side panel configuration prior to use;
Figs. 6A-C schematically show various exemplary embodiments of a disposable article comprising side panels provided with breakable bonds according to the disclosure; and
Figs. 7A-C schematically show a disposable article being unfolded from a folded side panel configuration in which the breakable bonds according to the disclosure is breaking.

### DETAILED DESCRIPTION OF EXEMPLARY EMBODIMENTS

Various aspects of the disclosure will now be described more fully hereinafter with reference to the accompanying drawings, in which exemplary embodiments of the disclosure are shown. The exemplary embodiments of the disclosure may, however, be embodied in many different forms and should not be construed as limited to the embodiment set forth herein; rather, these embodiments are provided for thoroughness and completeness. Like reference characters refer to like elements throughout the description.

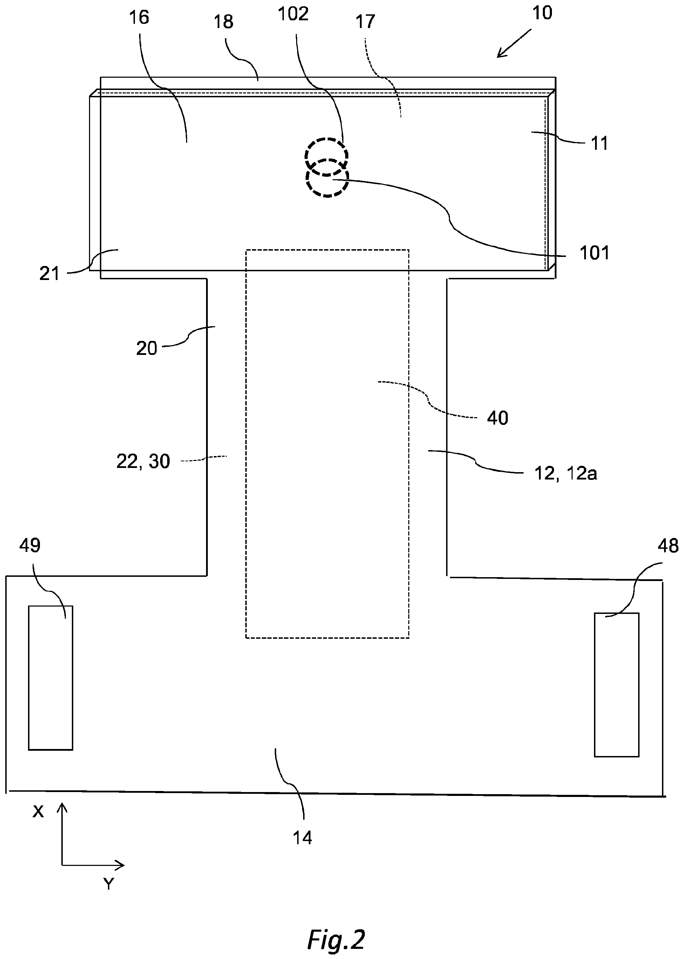

For purposes of the description herein the terms "rear," "front," "vertical," "horizontal,", "longitudinal,", "inner," "outer,", "exterior,", "interior", and derivatives thereof relate to the exemplary embodiment of the disclosure as oriented in, e.g., Fig. 2.

However, it is to be understood that the exemplary embodiments may assume various alternative orientations, except where expressly specified to the contrary. It is also to be understood that the examples illustrated in the attached drawings, and described in the following specification are simply exemplary embodiments. Hence, dimensions and other physical characteristics relating to the exemplary embodiments disclosed herein are not to be considered as limiting, unless the appended claims expressly state otherwise.

The disclosure mainly refers to disposable absorbent articles, which means articles that are not intended to be laundered or otherwise restored or reused as absorbent articles after use. The disclosure further relates to a disposable article of the belt type, a so-called belt article or belted article or a conventional open diaper type as further described herein.

The article comprises generally a chassis part. The article comprises transverse side panels connected to either the front or rear end part of the article's chassis part. The side panels may be an integral part of the chassis or separate parts attached to the chassis.

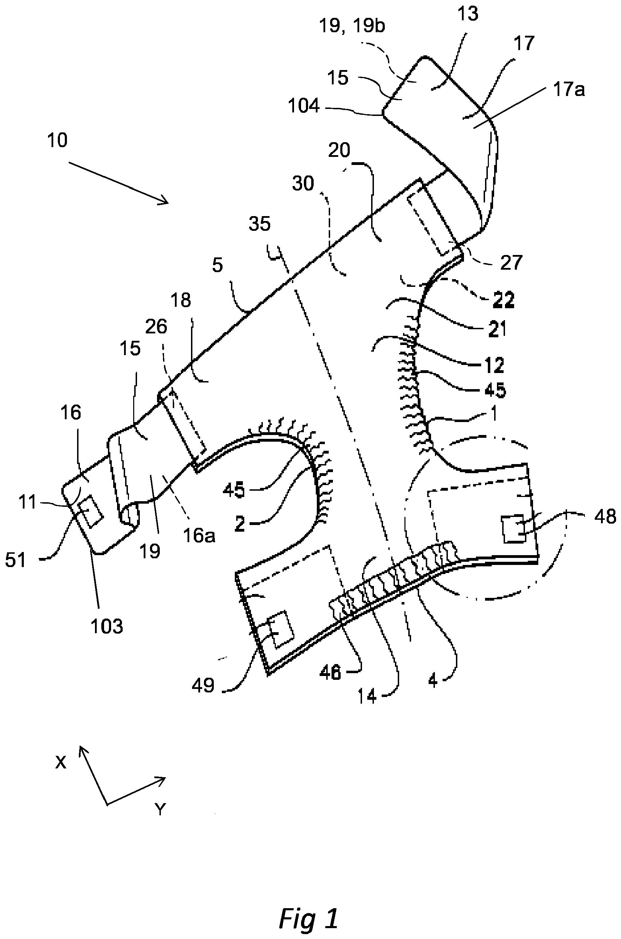

Figure 1 schematically shows some components of a belt type diaper article 10 according to an exemplary embodiment of the disclosure, in which the article 10 is shown from the side which is intended to face towards the wearer when in use.

Figure 2 schematically shows some components of an open diaper type article 10 according to an exemplary embodiment of the disclosure, in which the article 10 is shown from the side which is intended to face towards the wearer when in use.

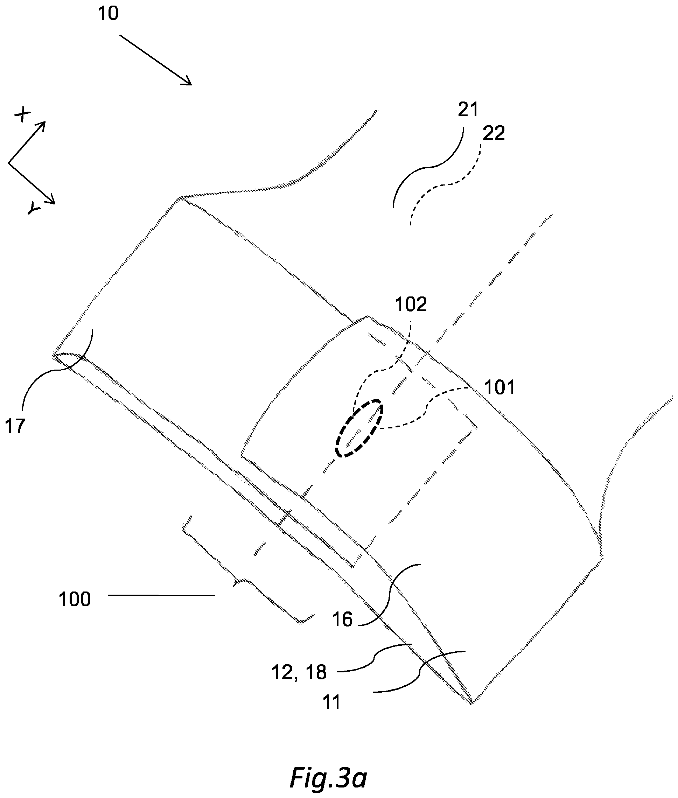

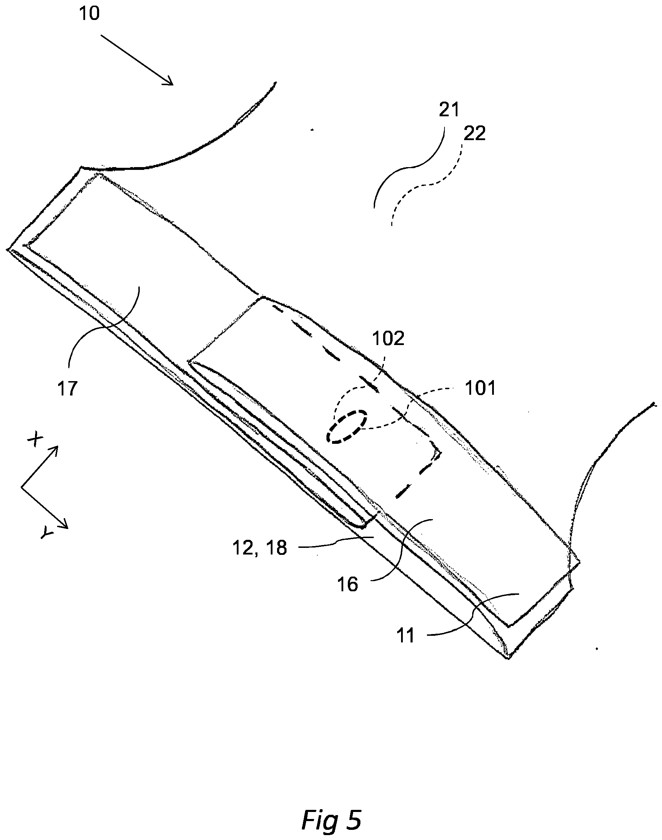

Further details of the exemplary embodiments of the disclosure are described herein in relation to figs. 3A-3B, 4, 5, 6A-C and 7A-C.

The article 10 comprises a chassis 12 and a side panel 16 and a second side panel 17. Further, the article 10 has a longitudinal direction X and a transverse direction Y. The chassis part 12 comprises a first covering layer 20. In other words, the article has a length extension in the longitudinal direction X and a width extension in the transverse direction Y. Moreover, the chassis 12 has a first end portion 18, a second end portion 14 and a central portion 12 extending therebetween.

Typically, the article comprises a body-oriented surface 21 facing the user during use and a garment-oriented surface 22 facing away from the user in use.

In the examples of Fig 1-2, the chassis includes the first covering layer 20, which forms the body-oriented surface 21 intended to face towards the user. In addition, the chassis also includes the garment-oriented surface 22 intended to face away from the user in use.

In other words, there is provided a disposable article 10, which comprises the chassis 12 having first and second end portions 18, 14 and a central portion 12 extending therebetween. The chassis also comprises the first covering layer 20 having the body-oriented surface 21 intended to face towards the user and the garment-oriented surface 22 intended to face away from the user in use.

In this exemplary embodiment, as shown in Figs. 1-2, the first end portion is a rear end part 18 and the second end portion is a front end part 14. However, the opposite may also be readily conceivable.

The article 10 further comprises a pair of side panels 16, 17 attached to the chassis 12 so that the first side panel 16 and the second side panel 17 extend on each side of the first end portion 18 of the chassis.

In the embodiments of Figs. 1-2 the side panels 16, 17 are arranged for securing to each other around a waist of a wearer of the article or to a third and fourth side panel respectively. The side panels 16, 17 have an exterior surface 16a, 17a, respectively. Each first and second side panel 16, 17 are connected to the chassis 12 in the rear end part 18, respectively. The first side panel 16 is joined to the longitudinal edge 2 and the second side panel 17 is joined to the opposing longitudinal edge 1.

Typically, although not strictly required, the side panels may be partly or entirely elastic.

The first and second side panels 16, 17 here have an inner surface 19 facing the wearer during use and an outer (exterior) surface 16a and 17a facing away from the wearer during use. Thus, the first side panel has an inner surface 19a facing the wearer during use. Likewise, the second side panel has an inner surface 19b facing the wearer during use. In the article shown in Figs. 1 and 2, the side panels (first side panel 16 and second side panel 17) extend in the transverse direction Y of the article. The side panels (first side panel 16 and second side panel 17) extend also in the longitudinal direction X.

The first side panel 16 is shown having a fastener 51 or a fastening component adapted to be attached to the second side panel 17 for securing the side panels to each other around the wearer of the article.

In this example, as shown in, e.g., Fig. 1, the first side panel 16 has a free end 11 which carries the fastener 51 adapted to be attached to an exterior surface 17a of the second side panel 17. The fastener is typically adapted to be attached in a refastenable manner, as is well-known in the art. Further, each one of fastener 51 and a mating fastening component arranged on another side panel to which the fastener 51 is to be attached may comprise a mechanical connector structure being capable of forming a mechanical interconnection with each other. One example of a mechanical connector structure is a hook and loop material. The loop material may be a nonwoven material of the absorbent article, such as a hook-to-textile mechanical connector solution, or the loop material may be a separate loop patch, laminated or attached to the absorbent article. Hereby, the fastener and the mating fastening component are mechanically connectable to form an interconnection between the first side panel and the second side panel for securing to each other around a wearer of the absorbent article. The interconnection may sometimes be referred to as refastenable connection.

The second end portion 14 of the chassis comprises a fastening device 48, 49 for securing the second end portion 14 of the chassis to the first and second side panels 16, 17 constituting a belt portion in this example so that the article assumes a pant-like shape with the belt portions forming a part of a waist portion of the pant. The second end portion 14 of the chassis is typically secured to the exterior surfaces 16a, 17a of the first side panel 16 and/or second side panel 17.

In other examples, such as the one shown in Fig. 2, the article 10 is provided with a second pair of side panels attached to the chassis 12 so that the third side panel and the fourth side panel extend on each side of the second end portion 14 of the chassis. The side panels 16, 17 of the first pair of side panels may carry a respective first and second fastener 51 adapted to be attached to the side panels of the second pair of side panels so that the article assumes a pant-like shape when in use.

Turning now to Fig. 2, the article 10 is depicted in a state in which the first and second side panels 16, 17 are folded over the body-oriented surface 21 to form a folded side panel configuration prior to use of the article 10. Hereby, the first side panel 16 is arranged on top of the second side panel 17. When the article comprises a first covering layer 20, the first and second side panels 16, 17 are folded over the body-oriented surface 21 of the first covering layer 20 to form a folded side panel configuration prior to use of the article.

Turning again to Fig. 2, the article is here depicted in its folded side panel configuration. As illustrated in Fig. 2, the second side panel 17 is arranged to overlap the chassis of the article, and the first side panel 16 is arranged to overlap the second side panel 17. In other words, the second side panel 17 is arranged to overlap the first end portion 18 of the chassis 12 and the first side panel 16 is arranged to overlap the second side panel 17. In this exemplary embodiment, the second side panel 17 is arranged to overlap an absorption portion of the article and the first side panel 16 is arranged to overlap the second side panel 17 entirely. Thus, when the first side panel 16 and the second side panel 17 are folded over the body-oriented surface 21 to form the folded side panel configuration prior to use of the article, the second side panel 17 is arranged to overlap the absorption portion 40 of the article and the first side panel 16 is arranged to overlap the second side panel 17. To this end, the fastener 51 on the free end 11 of the first side panel is releasably attached to the exterior surface 17a of the second side panel 17.

Further, the second side panel 17 is releasably bonded to the body-oriented surface 21 of the first covering layer 20 by a first breakable bond 101. In other words, when the first side panel 16 and the second side panel 17 are folded over the body-oriented surface 21 to form the folded side panel configuration prior to use of the article, the second side panel 17 is releasably bonded to the body-oriented surface 21 of the first covering layer 20 by the first breakable bond 101.

Analogously, the first side panel 16 is releasably bonded to the exterior surface 17a of the second side panel 17 by a second breakable bond 102. In other words, when the first side panel 16 and the second side panel 17 are folded over the body-oriented surface 21 to form the folded side panel configuration prior to use of the article, the first side panel 16 is releasably bonded to the exterior surface 17a of the second side panel 17 by the second breakable bond 102.

In the embodiment of Fig. 2, the third and fourth side panels are provided with a respective fastener 48, 49 such as to fasten the third and fourth side panel to the first and second side panel respectively so that the article assumes a pant-like shape when in use.

In some design variants, any one of the first and second side panels 16, 17 comprises an intermediate elastic region (not illustrated).

As mentioned above, the article 10 comprises a first covering layer 20, wherein the first covering layer comprises the body-oriented surface 21, intended to face towards the user when the article 10 is used. The article 10 also comprises the opposing garment-oriented surface 22.

In this example, the first covering layer is a first liquid-permeable covering layer, while a second covering layer is a second substantially liquid-impermeable covering layer 30. The first liquid-permeable covering layer 20 forms the body-oriented surface, whilst the second substantially liquid-impermeable covering layer 22 forms the garment-oriented surface.

Typically, although not strictly necessary, the first covering layer 20 of the article 10 has an hourglass shape. Other shapes such as a rectangular shape, for example, may also be conceivable in other design variants.

The first covering layer 20 can consist of any material suitable for the purpose. The most common covering materials for disposable articles are nonwoven textile materials, so-called nonwoven materials manufactured according to various methods. Not so common but also occurring is the use of covering layers 20 of woven material or net material. For example, a nonwoven material suitable as a covering layer 20 can be manufactured from synthetic fibers such as polyester or polypropylene, of natural fibers such as cotton fibers. A mix of synthetic and natural fibers also occurs. The manufacture of a nonwoven material consists of the two main stages of web formation and web bonding. Web formation means that a web is created of as even a surface weight as possible, wherein the web can be created by carding of natural or manmade staple fibers. The spunlaid technique is another web-forming technique, in which polymer material is extruded to thin continuous fiber threads on a continuous conveyor. The extruded thin fiber threads are randomized following extrusion in that they land on the continuous conveyor in a random manner. Web formation can take place in several different ways. A commonly occurring method is so-called thermal bonding, wherein a bonding pattern is melted into the fiber web in a roller nip where at least one of the hot rollers has a pattern. For web formation by means of thermal bonding to work, at least a certain portion of the fibers in the web must consist of synthetic fusible fibers. Occurring bonding patterns are dots of varying geometry, small rectangular or circular surfaces, floral patterns or the like. What is common to the methods described above, and also to other methods, is that not all the surface of the covering layer 20 is bonded, but between the bonding areas the covering layer has open areas comprising fiber threads that are fixed at one or both of their ends to the adjacent bonded areas.

Laminate consisting of two or more of the aforementioned possible covering materials also occur, such as a covering consisting of different materials in different parts of the surface. It also occurs that the covering layer 20 consists of an entirely or partially elastic material to give the article 10 a better fit when in use. The covering layer 20 also has a fastening device (e.g. a first and a second fastening device 48, 49) arranged at the respective longitudinal edge 1, 2 in the front end part 14 of the covering layer 20, i.e. connected to the two front corners of the covering layer 20. The fixing members 48, 49 are arranged on the side of the covering layer 20 that is intended to face towards the user when the article 10 is used. The fastening devices 48, 49 may typically comprise hook elements that are intended, on application of the article 10 to a user, to be connected detachably to loops arranged on the side of the side panels 16, 17 that are intended to face away from the user.

To improve the fit of the article 10, the longitudinal edges 1, 2 of the first covering layer 20 can be provided with leg elastics 45 arranged substantially in the longitudinal direction X of the article. The task of the leg elastics 45 includes improving the fit of the article, and making the article 10 more like textile multiple-use briefs/pants. Each respective leg elastic 45 can comprise one or more elastic threads that in an extended state have been joined to the covering layer 20 by gluing, ultrasonic welding, or the like. Alternatively, the respective leg elastic 45 can comprise elastic ribbon of foamed material, for example. The respective leg elastic 45 may be arranged on the side of the first covering layer 20 that is intended to face away from the user when in use.

The rear or front end parts 18, 14 of the article 10 can also be provided with so-called waist elastics 46 in the form of elastic elements arranged along a second transverse edge 5 or a first transverse edge 4 of the article 10 to give the article 10 a soft, flexible enclosure of the user's waist. In Fig. 1, only the front end part 14 of the article 10 is provided with a waist elastic 46. The waist elastic 46 comprises a thin strip of elastic foam material that is attached by glue to the side of the first covering layer 20 that is intended to face away from the user. The waist elastic 46 is applied in a stretched state to achieve a holding-together force that stretches the article 10 around the user's waist.

According to another aspect of the disclosure, the article 10 comprises a second covering layer 30 arranged on the side of the first covering layer 20 that is intended to face away from the user when in use. The second covering layer 30 has the same extension in the X/Y plane as the first covering layer 20. The second covering layer 30 is typically substantially liquid-impermeable, but other types of covering layers can also exist. The second covering layer 30 can comprise a range of different materials, but most frequently it comprises a thin, preferably liquid-impermeable plastic film, but it is also possible to use other types of substantially liquid-impermeable material such as nonwoven material that has been made substantially liquid-impermeable through plastic coating, for example, a liquid-impermeable foam layer, liquid-impermeable glue or the like. The second covering layer 30 can alternatively comprise a liquid-impermeable vapour-permeable material, a so-called breathable material.

Furthermore, laminates comprising at least one liquid-impermeable and typically breathable layer arranged against the garment-oriented surface 22 also exist as a second covering layer 30. These laminates usually consist of a liquid-impermeable material acting as a barrier against soaking through and a more textile-like material arranged on the side of the article 10 that is oriented away from the user on use. The outside of the article 10 is thereby more garment-like in use. The textile-like layer of the laminate normally consists of a nonwoven layer, but other textile or textile-like materials also exist. The first covering layer 20 and the second liquid-impermeable covering layer 30 can be joined to one another in several different ways. Examples of joining methods are gluing, thermal fusing, ultrasonic welding or the like. For articles 10 comprising a first covering layer 20 and a substantially liquid-impermeable second covering layer 30, it is suitable for the leg elastic 45 and the waist elastic 46 described above to be arranged between the two covering layers 20, 30. The first covering layer 20 may, in an article 10, have a low absorption capacity wherein smaller bodily secretions such as occasional drops of urine, for example, initially secreted menstruation fluid or similar.

According to one exemplary embodiment of the disclosure, as shown in Figs. 1-2, the article 10 has an absorption body 40 arranged between the first covering layer 20 and the second substantially liquid-impermeable covering layer 30. The first covering layer 20 of an article according to this example must be liquid-permeable. The article 10 in accordance with this example relates to a diaper intended to be used by an incontinent adult person or by a child who has not yet become continent. The absorption body 40 has substantially the same profile, but a smaller surface, than the first and second covering layers 20, 30. The two covering layers 20, 30 thus extend outside the edges of the absorption body 40 along the entire circumference of the absorption body 40. The absorption body 40 has, exactly like the covering layers 20, 30, a front and a rear end part and a narrower crotch part located between the end parts. Upon use of an article comprising the absorption body 40, the front part of the crotch part and the front end part principally act like a receiving area for urine, while the rear part of the crotch part and the rear end part act mainly as a receiving area for feces. The liquid-permeable first covering layer 20 and the substantially liquid-impermeable second covering layer 30 are connected to one another outside the absorption body 40 along its entire circumference. The absorption body 40 can be constructed from one or more layers of cellulose fluff pulp. The cellulose fluff pulp can be mixed with fibers or particles of a highly absorbent polymer material, so-called super absorbents, of the type that chemically binds large quantities of fluid on absorption with the formation of a fluid-holding gel. The absorption body 40 can also comprise highly absorbent polymer material arranged in a layer inside the absorption body or connected to the surface or surfaces of the absorption body. The absorption body 40 can further include further components for improving the properties of the absorption body 40. Examples of such components are binding fibers, various types of fluiddispersing layers or fibers, dimensionally stabilizing components, reinforcing fibers or the like.

Further, in all exemplary embodiments described herein, and in other possible exemplary embodiments, a part of any one of the first and second side panels extending from the first end portion of the article is typically permanently attached to the first end portion.

As mentioned above, the article is typically a disposable absorbent article such as a diaper and an incontinence guard. Further, the article 10 in this example comprises the absorption body 40 arranged between the first liquid-permeable covering layer 20 and the second substantially liquid-impermeable covering layer 30.

Fig. 3A shows another exemplary embodiment of the disclosure, in which the article 10 is depicted in a state in which the first and second side panels 16, 17 are folded over the body-oriented surface 21 to form the folded side panel configuration prior to use of the article 10. Hereby, the first side panel 16 is arranged on top of the second side panel 17. When the article comprises a first covering layer 20, the first and second side panels 16, 17 are folded over the body-oriented surface 21 of the first covering layer 20 to form the folded side panel configuration prior to use of the article. The side panels of Fig. 3A are shorter than the side panels depicted in Fig. 1-2, otherwise the properties of the article are the same. According to Fig. 3A the first breakable bond 101 and the second breakable bond 102 are arranged in the overlap area 100, and overlap each other. In other words, the second breakable bond 102 is arranged on top of the first breakable bond when the article is in the folded side panel state. The first and second breakable bonds 101, 102 are arranged along the longitudinal central line of the article 10, as depicted by the dashed line in Fig. 3A. The breakable bonds 101, 102 are centrally arranged in the longitudinal direction X on the respective side panels 16, 17. In Fig. 3A the breakable bonds are a separate part of the respective side panels 16, 17 and depicted as adhesive spots.

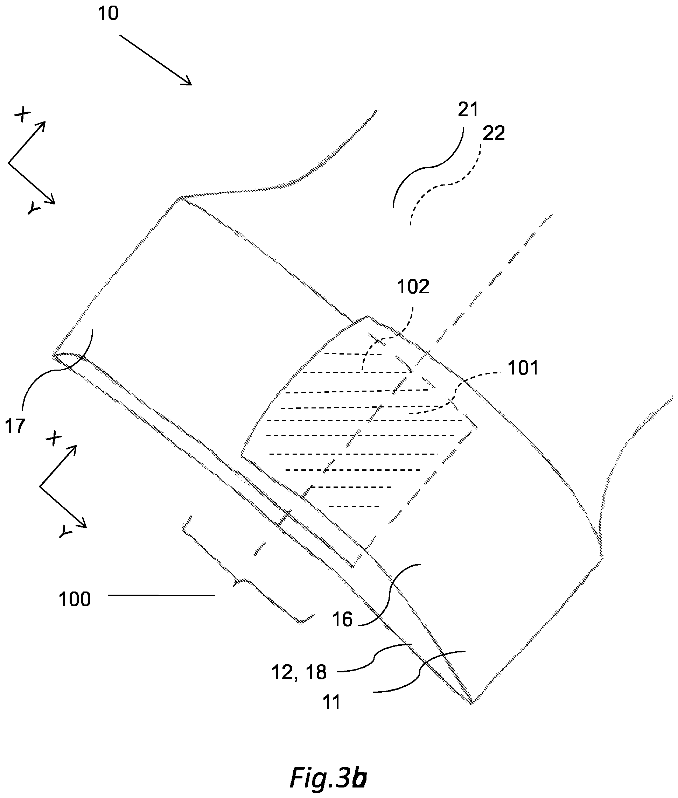

In Fig. 3B, the first breakable bond 101 forms a pattern over the entire part of the second side panel 17 overlapping the first side panel 16 in the overlap area 100. Similarly, the second breakable bond 102 forms a pattern over the entire part of the first side panel 16 arranged on top of the second side panel 17 in the overlap area 100 when the article is in the folded side panel configuration. In this example, the breakable bond is an integral part of the first and second side panels respectively, and may for example be formed by ultrasonic welding or thermobonding.

Other embodiments are possible where the breaking bonds are provided with randomly selected or varied patterns.

The breakable bonds are typically centrally arranged in the longitudinal direction X of the of the side panel. However, variants are conceivable wherein any desired asymmetry of the arrangement of the breakable bonds is possible.

In view of the above disclosure explaining how to provide the breakable bonds on the side panels, it will be understood that the article is provided with improved ability in terms of allowing a user of the article to release the side panels and unfold the side panels from their folded configuration in a simple motion.

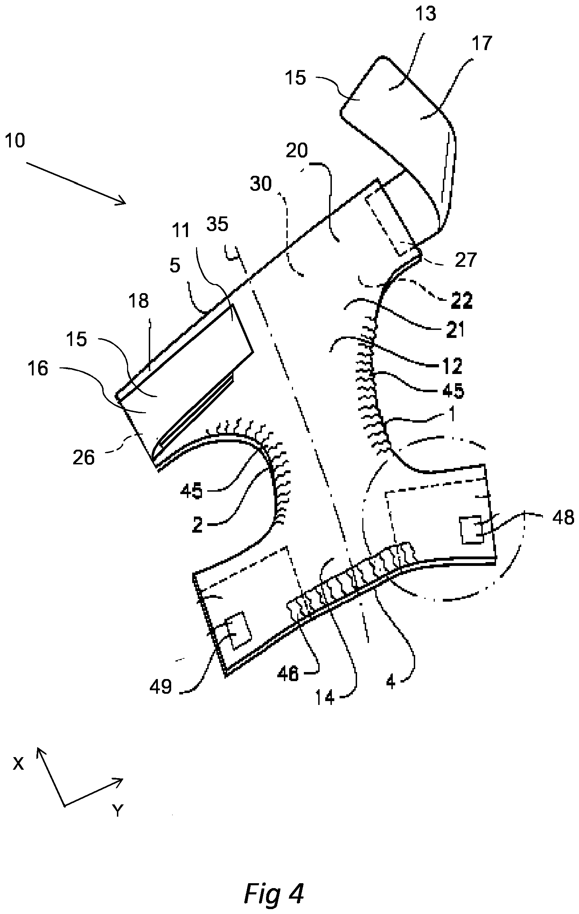

Fig. 4 shows another exemplary embodiment of the disclosure, in which at least one of the side panels is folded in order to wrap up a length of the at least one side panel. As mentioned above, the article 10 has a body-oriented surface 21 facing the user during use and a garment-oriented surface 22 facing away from the user in use. The article further comprises a pair of side panels 16, 17 attached to the chassis so that a first side panel 16 and a second side panel 17 extend on each side of the first end portion 18 of the chassis. In this example, the folded side panel configuration is formed by having the second side panel 17 folded over the body-oriented surface 21, although the second side panel is illustrated in Fig. 4 in an unfolded position (or loose hanging position).

In addition, as is clearly shown in Fig. 4, the first side panel 16 is folded in order to wrap up a length of the side panel 16. Typically, although not strictly required (and not shown), the second side panel 17 is also folded in order to wrap up a length of the side panel 17. In this exemplary embodiment, the first belt portion is folded over the body-oriented surface 21 in an accordion-like fashion to form a Z-shape, as seen in a cross section taken along the transverse direction Y. The accordion-like fold may however also be formed in several different manners, for example being folded in a Z-shape, M-shape, W-shape or the like, over the body-oriented surface. In this example, the extension of the folded side panel is less than half the transverse length of the chassis, as shown in Fig. 4. However, the extension of the folded side panel may be varied depending on type of article, type of side panel, type of fold and/or desired length of the side panel when being unfolded. It is appreciated that an even larger amount of folds can be used in order to fold a desired amount of material of a side panel, in particular a belt side panel. For instance, a first fold may be a hook protection fold, wherein a portion of the backsheet nonwoven side panel is folded over, thereby at least partly covering and protecting the hooks when the absorbent article is in a state prior to being worn by a user, such as when provided in a product package during shipping (not shown). The hook protection fold keep the hooks from attaching to other parts of the absorbent article during manufacturing, packaging or transport.

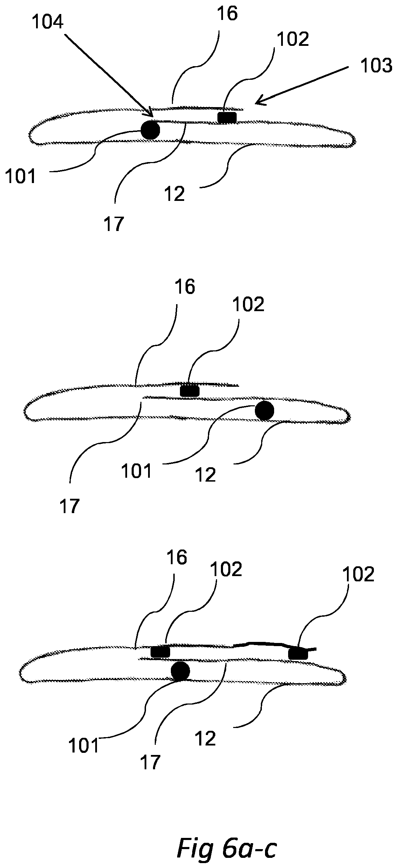

Figs. 6A-C illustrate further exemplary embodiments of the breakable bond arrangements in the folded side panel configuration prior to use of the article 10. The article is in the folded side panel configuration in Figs. 6A-C. The distal edge 103 of the first side panel 16 is preferably free such that it is grippable by a user intending to release the breakable bonds to unfold the folded side panel configuration.

In Fig. 6A the first breakable bond 101 is arranged adjacent the distal edge 104 of the second side panel 17. The second breakable bond 102 is arranged adjacent the distal edge 103 of the first side panel 16.

In Fig. 6B the second breakable bond 102 is arranged along the longitudinal central line of the end portion of the chassis 12 of the article 10. The first breakable bond 101 is arranged adjacent the fold line of the second side panel 17, and is not arranged in the overlap area 100.

The first side panel 16 depicted in Fig. 6C is longer than the second side panel 17. The first breakable bond 101 is arranged centrally in transverse direction Y of the chassis 12. The second breakable bond 102 is arranged to cover several parts of the part of the first side panel 16 arranged on top of, and thereby overlapping, the second side panel 17.

In one design option, although not illustrated, the second side panel 17 can likewise be folded over the garment-oriented surface 22 (when the side panels 16, 17 are in the folded side panel configuration prior to use).

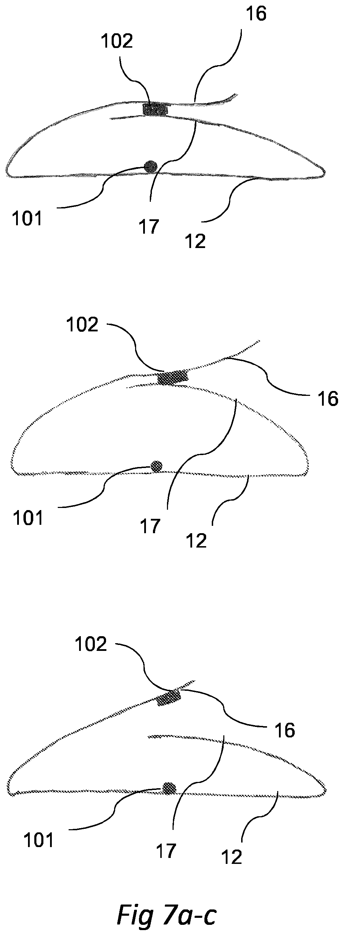

Figs. 7A-C illustrate the sequential release of the breakable bonds 101, 102 once the first side panel 16 is being pulled, preferably by its free distal edge 103. The first breakable bond 101 between the second side panel 17 and the chassis 12 is weaker in peel strength than the second breakable bond 102 between the second side panel 17 and the first side panel 16 arranged in top of the second side panel 17. Due to the weaker bond strength, the first breakable bond will break first, thereby releasing the bonded second and first side panels 16, 17 from the chassis 12. The second breakable bond 102 is still intact in Figs. 7A-B. Continuous pulling of the first side panel will eventually also release the second breakable bond 102, and thereby provide two loose side panels 16, 17 as illustrated in Fig. 7C. In one single grip, and by one single pulling motion, both the side panels 16, 17 can be sequentially released; thereby facilitating the unwrapping of the article from a folded side panel configuration to a ready-to-use configuration. The side panels 16, 17 in Fig. 7C are readily available for use.

As mentioned above, in all exemplary embodiments described herein, and in other possible exemplary embodiments, a part of any one of the first and second side panels extending from the first end portion of the article is typically permanently attached to the first end portion. In other words, an end portion 26 of the first side panel 16 is here permanently attached to the first end portion 18 of the chassis. Analogously, an end portion 27 of the second side panel 17 is here permanently attached to the first end portion 18 of the chassis. Also, as mentioned above, the article is typically a disposable absorbent article such as a diaper and an incontinence guard. Further, the article 10 in the exemplary embodiments described above comprises the absorption body 40 arranged between the first liquid-permeable covering layer 20 and the second substantially liquid-impermeable covering layer 30.

The term "nonwoven" is applied to a wide range of products which in term of their properties are located between the groups of paper and cardboard on the one hand and textiles on the other hand. As regards nonwovens, a large number of extremely varied production processes are used, such as airlaid, wetlaid, spunlaced, spunbond, meltblown techniques etc. The fibers may be in the form of endless fiber or fibers prefabricated with an endless length, as synthetic fibers produced in situ or in the form of staple fibers. Alternatively, they may be made from natural fibers or from blends of synthetic fibers and natural fibers.

By "absorbent article" is meant an article that absorbs or is adapted to absorb bodily fluids, such as urine and/or blood.

The disclosure also covers all conceivable combinations of the described aspects, variants, alternatives and exemplary embodiments of the disclosure.

Furthermore, the disclosure is not limited to the aforesaid aspects or examples, but is naturally applicable to other aspects and exemplary embodiments within the scope of the following claims.

Reference signs mentioned in the claims should not be seen as limiting the extent of the matter protected by the claims, and their sole function is to make claims easier to understand.

### DESCRIPTION OF TEST METHOD

Measuring the strength of the first and second bonds, the peel strength of the bonds, may be done using a conventional tensile tester (available e.g. from Instron, 825 University Avenue, Norwood, MA 02062 USA or Lloyd corporations).

The width of the upper and lower clamps of the tensile tester should be wide enough so that they can grab and clamp over the whole width of the side panels thereby ensuring that an even tensile force is applied to the side panels.

The whole waist part of the diaper should be cut off, to a width corresponding to the width of the side panels, when the side panels are of essentially the same width. Thereafter, first measure the strength of the second breakable bond (i.e. the panel to panel bond) of the disposable article.

Insert the distal end of the first side panel into the upper (movable) clamp, and insert the chassis section of the disposable article, the longitudinal edge of the chassis arranged adjacent the distal end of the first side panel and which longitudinal edge to which the second side panel is fixedly attached to, into the bottom (fixed) clamp. The gauge length (the distance between the clamps) can suitably be 25 mm or less, prior to operating the tensile tester for measuring the strength of a bond.

When starting the test, the upper clamp should be raised with a crosshead speed of 500 mm/min. Gently hold or support the sample (the not yet separated section) at a 90° angle (i.e. perpendicular to the vertical line between the clamps) during the test. End the test when all bonds, or in the case of a single bond between the side panels - the second breakable bond, between the first side panel and the second side panel - have been completely separated.

Next measure the first breakable bond (i.e. panel to chassis) using the same procedure, as when measuring the second breakable bond(s). Insert the distal end of the second side panel into the upper (movable) clamp, and insert the chassis section, the longitudinal edge of the chassis arranged adjacent the distal end of the second side panel and which longitudinal edge to which the first side panel is fixedly attached to of the disposable article, into the bottom (fixed) clamp. Gently hold or support the sample (the not yet separated section) at a 90° angle (i.e. perpendicular to the vertical line between the clamps) during the test, when the gauge length (the distance between the clamps) is increased. End the test when all bonds, or in the case of a single bond between the second side panel and the chassis - the first breakable bond, between the second side panel and the chassis - have been completely separated.

The peel strength equals the maximum value in Newtons (N), measured during testing using the tensile tester, for the respective panel to panel and panel to chassis separations. The reported value should be an average from at least five different diapers.

The testing method is applicable to embodiments comprising: a single first and/or breakable bond, as well as a plurality of first and/or second breakable bonds.

## Claims

1. A disposable article (10) comprising a chassis (12) having first and second end portions (18, 14) and a central portion (12a) extending therebetween, said article further comprising a first pair of side panels (16, 17) extending on each side of the first end portion (18) of the chassis (12) and said first and second side panels (16, 17) being folded over said chassis (12), to form a folded side panel configuration prior to use of said article (10), and said first side panel (16) being arranged at least partly on top of the second side panel (17) to form an overlap area (100) in the folded side panel configuration,
wherein the second side panel (17) is bonded to the chassis (12) by a first breakable bond (101) in said folded side panel configuration and the first side panel (16) is bonded to the second side panel (17) by a second breakable bond (102)
in said overlap area (100), and
wherein the first breakable bond (101) has a weaker peel strength than the second breakable bond (102) when the peel strength is measured according to a test method as described in the description.

2. Disposable article (10) according to claim 1, wherein said article (10) having a body-oriented surface (21) facing the user during use and a garment-oriented surface (22) facing away from the user in use, and wherein the second side panel is folded over the body-oriented surface (21) or the garment-oriented surface (22) when said article is in said folded side panel configuration.

3. Disposable article (10) according to any one of the preceding claims, wherein said first and second side panel (16, 17) comprises distal edges (103, 104) respectively, and each one of said distal edges (103, 104) of said first and second side panels (16, 17) being folded over and beyond a longitudinal central line (35) when said article is in said folded side panel configuration prior to use such that the overlap area (100) is centrally arranged along the longitudinal central line of the disposable article (10).

4. Disposable article (10) according to any one of the preceding claims, wherein the first breakable bond (101) is arranged in the overlap area (100) when said article (10) is in said folded side panel configuration and/or the second breakable bond (102) is arranged on top of the first breakable bond (101) when the article is in said folded side panel configuration prior to use and/or wherein the breakable bonds (101, 102) are arranged adjacent or on the longitudinal central line (35) of the disposable article (10).

5. Disposable article (10) according to any one of the preceding claims, wherein the first breakable bond (101) is arranged in a pattern that covers the entire part of the second side panel (17) in the overlap area and/or the second breakable bond (102) is arranged in a pattern that covers the entire part of the first side panel (16) in the overlap area.

6. Disposable article (10) according to any one of the preceding claims, wherein a force of 4-6 N is required to rupture the first breakable bond (101) and a force 7-14 N is required to rupture the second breakable bond (102), and/or wherein the force required for rupturing the first breakable bond (101) may be between 20-80% of the force required for rupturing the second breakable bond (102).

7. Disposable article (10) according to any one of the preceding claims, wherein the first breakable bond (101) is an integral part of the second side panel (17), and/or the second breakable bond (102) is an integral part of the first side panel (16) or wherein the first breakable bond (101) is a separate part of the second side panel (17), and/or the second breakable bond (102) is a separate part of the first side panel (16).

8. Disposable article (10) according to any one of the preceding claims, wherein the at least one side panel is folded in an accordion-like fashion, for example folded in a Z-shape, M-shape, W-shape or the like, over said body-oriented surface (21) or over said garment-oriented surface (22).

9. Disposable article (10) according to any one of the preceding claims, wherein any one of the first and second side panels (16, 17) comprises an intermediate elastic region.

10. Disposable article (10) according to any one of the preceding claims, wherein the disposable article (10) is a disposable absorbent article such as a diaper, a belted-diaper and an incontinence guard.

11. Disposable article (10) according to any one of the preceding claims, wherein the disposable article (10) has an absorption body (40) arranged between a first liquid-permeable covering layer (20), which may form the body-oriented surface (21), and a second substantially liquid-impermeable covering layer (30), which may form the garment-oriented surface (22).

12. Disposable article (10) according to any one of the preceding claims, wherein said article comprises a second pair of side panels attached to the chassis (12) so that a third side panel and a fourth side panel extend on each side of the second end portion (14) of the chassis (12).

13. Disposable article (10) according to claim 12, wherein the side panels (16, 17) of the first pair of side panels carries a respective first and second fastener (51) adapted to be attached to the side panels of said second pair of side panels so that said article assumes a pant-like shape when in use.

14. Disposable article (10) according to claim 13, wherein the first and second fasteners are arranged on the distal edge (103, 104) of said respective first and second side panel (16, 17), and wherein the distal edge of the first side panel (16) is folded over the first fastener such as to cover the first fastener in said folded side panel configuration, and the distal edge of the second side panel (17) is folded over the second fastener such as to cover the second fastener in said folded side panel configuration, and wherein the first breakable bond (101) is arranged on the part of the first side panel (16) that covers the first fastener and the second breakable bond (102) is arranged on the part of the second side panel (17) that covers the second fastener.

15. Disposable article (10) according any one of the claims 1-11, wherein any one of the side panels (16, 17) of the first pair of side panels carries a fastener (51) adapted to be attached to an exterior surface of the other of the side panels of the first pair of side panels, and wherein said second end portion (14) of the chassis comprises a fastening device (48, 49) for securing the second end portion (14) of the chassis (12) to one of the side panels so that said article assumes a pant-like shape with the side panels forming a part of a waist portion of the pant when in use.

## Patentansprüche

1. Ein Einwegartikel (10), der ein Chassis (12) mit ersten und zweiten Endteilen (18, 14) und einem sich dazwischen erstreckenden mittleren Teil (12a) aufweist, wobei der Artikel weiterhin ein erstes Paar von Seitenpaneelen (16, 17), die sich auf beiden Seiten des ersten Endteils (18) des Chassis (12) erstrecken, aufweist, wobei die ersten und zweiten Seitenpaneele (16, 17) über das Chassis (12) gefaltet werden, um eine gefaltete Seitenpaneelkonfiguration vor der Verwendung des Artikels (10) zu bilden, und wobei das erste Seitenpaneel (16) wenigstens teilweise über dem zweiten Seitenpaneel (17) angeordnet ist, um einen Überlappungsbereich (100) in der gefalteten Seitenpaneelkonfiguration zu bilden,
wobei das zweite Seitenpaneel (17) mit dem Chassis (12) mittels einer ersten reißbaren Verbindung (101) in der gefalteten Seitenpaneelkonfiguration verbunden ist und das erste Seitenpaneel (16) mit dem zweiten Seitenpaneel (17) mittels einer zweiten reißbaren Verbindung (102) in dem Überlappungsbereich (100) verbunden ist, und
wobei die erste reißbare Verbindung (101) eine schwächere Abziehfestigkeit als die zweite reißbare Verbindung (102) aufweist, wenn die Abziehfestigkeit mittels einer Testmethode wie in der Beschreibung beschrieben gemessen wird.

2. Einwegartikel (10) nach Anspruch 1, wobei der Artikel (10) eine körperzugewandte Fläche (21), die während der Verwendung dem Benutzer zugewandt ist, und eine kleidungszugewandte Fläche (22), die während der Verwendung von dem Benutzer abgewandt ist, aufweist und wobei das zweite Seitenpaneel über die körperzugewandte Fläche (21) oder die kleidungszugewandte Fläche (22) gefaltet ist, wenn sich der Artikel in der gefalteten Seitenpaneelkonfiguration befindet.

3. Einwegartikel (10) nach einem der vorstehenden Ansprüche, wobei die ersten und zweiten Seitenpaneele (16, 17) jeweils ferne Kanten (103, 104) aufweisen und wobei jede der fernen Kanten (103, 104) der ersten und zweiten Seitenpaneele (16, 17) über eine Längsmittenlinie (35) hinaus gefaltet ist, wenn sich der Artikel in der gefalteten Seitenpaneelkonfiguration vor der Verwendung befindet, sodass der Überlappungsbereich (100) mittig entlang der Längsmittenlinie des Wegwerfartikels (10) angeordnet ist.

4. Einwegartikel (10) nach einem der vorstehenden Ansprüche, wobei die erste reißbare Verbindung (101) in dem Überlappungsbereich (100) angeordnet ist, wenn sich der Artikel (10) in der gefalteten Seitenpaneelkonfiguration befindet, und/oder die zweite reißbare Verbindung (102) über der ersten reißbaren Verbindung (101) angeordnet ist, wenn sich der Artikel in der gefalteten Seitenpaneelkonfiguration vor der Verwendung befindet, und/oder wobei die reißbaren Verbindungen (101, 102) neben oder auf der Längsmittenlinie (35) des Einwegartikels (10) angeordnet sind.

5. Einwegartikel (10) nach einem der vorstehenden Ansprüche, wobei die erste reißbare Verbindung (101) in einem Muster angeordnet ist, das den gesamten Teil des zweiten Seitenpaneels (17) in dem Überlappungsbereich bedeckt, und/oder die zweite reißbare Verbindung (102) in einem Muster angeordnet ist, das den gesamten Teil des ersten Seitenpaneels (16) in dem Überlappungsbereich bedeckt.

6. Einwegartikel (10) nach einem der vorstehenden Ansprüche, wobei eine Kraft von 4-6 N erforderlich ist, um die erste reißbare Verbindung (101) zu reißen, und eine Kraft von 7-14 N erforderlich ist, um die zweite reißbare Verbindung (102) zu reißen, und/oder wobei die für das Reißen der ersten reißbaren Verbindung (101) erforderliche Kraft zwischen 20-80% der für das Reißen der zweiten reißbaren Verbindung (102) erforderlichen Kraft betragen kann.

7. Einwegartikel (10) nach einem der vorstehenden Ansprüche, wobei die erste reißbare Verbindung (101) ein integrierter Teil des zweiten Seitenpaneels (17) ist und/oder die zweite reißbare Verbindung (102) ein integrierter Teil des ersten Seitenpaneels (16) ist oder wobei die erste reißbare Verbindung (101) ein separater Teil des zweiten Seitenpaneels (17) ist und/oder die zweite reißbare Verbindung (102) ein separater Teil des ersten Seitenpaneels (16) ist.

8. Einwegartikel (10) nach einem der vorstehenden Ansprüche, wobei das wenigstens eine Seitenpaneel akkordeonartig zum Beispiel in einer Z-Form, einer M-Form, einer W-Form oder ähnlichem über die körperzugewandte Fläche (21) oder über die kleidungszugewandte Fläche (22) gefaltet ist.

9. Einwegartikel (10) nach einem der vorstehenden Ansprüche, wobei eines der ersten und zweiten Seitenpaneele (16, 17) einen mittleren elastischen Bereich aufweist.

10. Einwegartikel (10) nach einem der vorstehenden Ansprüche, wobei der Einwegartikel (10) ein absorbierender Einwegartikel wie etwa eine Windel, eine mit einem Gürtel versehene Windel oder ein Inkontinenzschutzartikel ist.

11. Einwegartikel (10) nach einem der vorstehenden Ansprüche, wobei der Einwegartikel (10) einen Absorptionskörper (40) aufweist, der zwischen einer ersten für Flüssigkeiten durchlässigen Deckschicht (20), die die körperzugewandte Fläche (21) bilden kann, und einer zweiten für Flüssigkeiten im Wesentlichen undurchlässigen Deckschicht (30), die die kleidungszugewandte Fläche (22) bilden kann, angeordnet ist.

12. Einwegartikel (10) nach einem der vorstehenden Ansprüche, wobei der Artikel ein zweites Paar von Seitenpaneelen aufweist, die an dem Chassis (12) befestigt sind, sodass sich ein drittes Seitenpaneel und ein viertes Seitenpaneel auf jeder Seite des zweiten Endteils (14) des Chassis (12) erstrecken.

13. Einwegartikel (10) nach Anspruch 12, wobei die Seitenpaneele (16, 17) des ersten Paars von Seitenpaneelen entsprechende erste und zweite Befestigungselemente (51) tragen, die ausgebildet sind, um an den Seitenpaneelen des zweiten Paars von Seitenpaneelen befestigt zu werden, sodass der Artikel in der Verwendung eine hosenartige Form annimmt.

14. Einwegartikel (10) nach Anspruch 13, wobei die ersten und zweiten Befestigungselemente an den fernen Kanten (103, 104) der entsprechenden ersten und zweiten Seitenpaneele (16, 17) angeordnet sind und wobei die ferne Kante des ersten Seitenpaneels (16) über das erste Befestigungselement gefaltet ist, um das erste Befestigungselement in der gefalteten Seitenpaneelkonfiguration zu bedecken, und wobei die ferne Kante des zweiten Seitenpaneels (17) über das zweite Befestigungselement gefaltet ist, um das zweite Befestigungselement in der gefalteten Seitenpaneelkonfiguration zu bedecken, und wobei die erste reißbare Verbindung (101) an dem Teil des ersten Seitenpaneels (16), der das erste Befestigungselement bedeckt, angeordnet ist, und die zweite reißbare Verbindung (102) an dem Teil des zweiten Seitenpaneels (17), der das zweite Befestigungselement bedeckt, angeordnet ist.

15. Einwegartikel (10) nach einem der Ansprüche 1 bis 11 wobei eines der Seitenpaneele (16, 17) des ersten Paars von Seitenpaneelen ein Befestigungselement (51) trägt, das ausgebildet ist, um an einer Außenfläche des anderen Seitenpaneels aus dem ersten Paar von Seitenpaneelen befestigt zu werden, und wobei der zweite Endteil (14) des Chassis eine Befestigungseinrichtung (48, 49) für das Fixieren des zweiten Endteils (14) des Chassis (12) an einem der Seitenpaneele aufweist, sodass der Artikel eine hosenartige Form annimmt und die Seitenpaneele einen Teil eines Taillenteils der Hose in der Verwendung bilden.

## Revendications

1. Article jetable (10) comprenant un châssis (12) présentant des première et seconde parties d'extrémité (18, 14) et une partie centrale (12a) s'étendant entre celles-ci, ledit article comprenant en outre une première paire de panneaux latéraux (16, 17) s'étendant de chaque côté de la première partie d'extrémité (18) du châssis (12) et lesdits premier et deuxième panneaux latéraux (16, 17) étant pliés sur ledit châssis (12), pour former une configuration de panneau latéral plié avant utilisation dudit article (10), et ledit premier panneau latéral (16) étant agencé au moins partiellement sur le dessus du deuxième panneau latéral (17) pour former une zone de chevauchement (100) dans la configuration de panneau latéral plié,
dans lequel le deuxième panneau latéral (17) est lié au châssis (12) par une première liaison cassable (101) dans ladite configuration de panneau latéral plié et le premier panneau latéral (16) est fixé au deuxième panneau latéral (17) par une seconde liaison cassable (102) dans ladite zone de chevauchement (100), et
dans lequel la première liaison cassable (101) présente une résistance au pelage plus faible que la seconde liaison cassable (102) lorsque la résistance au pelage est mesurée selon un procédé de test tel que décrit dans la description.

2. Article jetable (10) selon la revendication 1, dans lequel ledit article (10) présente une surface orientée corps (21) faisant face à l'utilisateur en utilisation et une surface orientée vêtement (22) faisant face à l'opposé de l'utilisateur en utilisation, et dans lequel le deuxième panneau latéral est plié sur la surface orientée corps (21) ou la surface orientée vêtement (22) lorsque ledit article est dans ladite configuration de panneau latéral plié.

3. Article jetable (10) selon l'une quelconque des revendications précédentes, dans lequel lesdits premier et deuxième panneaux latéraux (16, 17) comprennent respectivement des bords distaux (103, 104), et chacun desdits bords distaux (103, 104) desdits premier et deuxième panneaux latéraux (16, 17) étant plié sur et au-delà d'une ligne centrale longitudinale (35) lorsque ledit article est dans ladite configuration de panneau latéral plié avant utilisation de telle sorte que la zone de chevauchement (100) soit agencée de manière centrale le long de la ligne centrale longitudinale de l'article jetable (10).

4. Article jetable (10) selon l'une quelconque des revendications précédentes, dans lequel la première liaison cassable (101) est agencée dans la zone de chevauchement (100) lorsque ledit article (10) se trouve dans ladite configuration de panneau latéral plié et/ou la seconde liaison cassable (102) est agencée sur le dessus de la première liaison cassable (101) lorsque l'article se trouve dans ladite configuration de panneau latéral plié avant utilisation et/ou dans lequel les liaisons cassables (101, 102) sont agencées de manière adjacente ou sur la ligne centrale longitudinale (35) de l'article jetable (10).

5. Article jetable (10) selon l'une quelconque des revendications précédentes, dans lequel la première liaison cassable (101) est agencée selon un motif qui recouvre la totalité de la partie du deuxième panneau latéral (17) dans la zone de chevauchement et/ou la seconde liaison cassable (102) est agencée selon un motif qui recouvre la totalité de la partie du premier panneau latéral (16) dans la zone de chevauchement.

6. Article jetable (10) selon l'une quelconque des revendications précédentes, dans lequel une force de 4 à 6 N est requise pour rompre la première liaison cassable (101) et une force de 7 à 14 N est requise pour rompre la seconde liaison cassable (102), et/ou dans lequel la force requise pour rompre la première liaison cassable (101) peut être comprise entre 20 et 80 % de la force requise pour rompre la seconde liaison cassable (102).

7. Article jetable (10) selon l'une quelconque des revendications précédentes, dans lequel la première liaison cassable (101) fait partie intégrante du deuxième panneau latéral (17), et/ou la seconde liaison cassable (102) fait partie intégrante du premier panneau latéral (16) ou dans lequel la première liaison cassable (101) est une partie séparée du deuxième panneau latéral (17), et/ou la seconde liaison cassable (102) est une partie séparée du premier panneau latéral (16).

8. Article jetable (10) selon l'une quelconque des revendications précédentes, dans lequel ledit au moins un panneau latéral est plié en accordéon, par exemple plié en forme de Z, en forme de M, en forme de W ou analogue, sur ladite surface orientée corps (21) ou sur ladite surface orientée vêtement (22).

9. Article jetable (10) selon l'une quelconque des revendications précédentes, dans lequel l'un quelconque des premier et deuxième panneaux latéraux (16, 17) comprend une région élastique intermédiaire.

10. Article jetable (10) selon l'une quelconque des revendications précédentes, dans lequel l'article jetable (10) est un article absorbant jetable tel qu'une couche, une couche-culotte et une protection contre l'incontinence.

11. Article jetable (10) selon l'une quelconque des revendications précédentes, dans lequel l'article jetable (10) présente un corps absorbant (40) agencé entre une première couche de revêtement perméable aux liquides (20), qui peut former la surface orientée corps (21), et une seconde couche de revêtement sensiblement imperméable aux liquides (30), qui peut former la surface orientée vêtement (22).

12. Article jetable (10) selon l'une quelconque des revendications précédentes, dans lequel ledit article comprend une seconde paire de panneaux latéraux fixés au châssis (12) de telle sorte qu'un troisième panneau latéral et un quatrième panneau latéral s'étendent de chaque côté de la seconde partie d'extrémité (14) du châssis (12).

13. Article jetable (10) selon la revendication 12, dans lequel les panneaux latéraux (16, 17) de la première paire de panneaux latéraux portent des première et seconde fixations (51) respectives adaptées pour être fixées aux panneaux latéraux de ladite seconde paire de panneaux latéraux de telle sorte que ledit article adopte une forme analogue à une culotte en utilisation.

14. Article jetable (10) selon la revendication 13, dans lequel les première et seconde fixations sont agencées sur le bord distal (103, 104) desdits premier et deuxième panneaux latéraux (16, 17) respectifs, et dans lequel le bord distal du premier panneau latéral (16) est plié sur la première fixation de manière à recouvrir la première fixation dans ladite configuration de panneau latéral plié, et le bord distal du deuxième panneau latéral (17) est plié sur la seconde fixation de manière à recouvrir la seconde fixation dans ladite configuration de panneau latéral plié, et dans lequel la première liaison cassable (101) est agencée sur la partie du premier panneau latéral (16) qui recouvre la première fixation et la seconde liaison cassable (102) est agencée sur la partie du deuxième panneau latéral (17) qui recouvre la seconde fixation.

15. Article jetable (10) selon l'une quelconque des revendications 1 à 11, dans lequel l'un quelconque des panneaux latéraux (16, 17) de la première paire de panneaux latéraux porte une fixation (51) adaptée pour être fixée à une surface extérieure de l'autre des panneaux latéraux de la première paire de panneaux latéraux, et dans lequel ladite seconde partie d'extrémité (14) du châssis comprend un dispositif de fixation (48, 49) pour fixer la seconde partie d'extrémité (14) du châssis (12) à l'un des panneaux latéraux de telle sorte que ledit article adopte une forme analogue à celle d'une culotte, les panneaux latéraux formant une partie d'une partie de taille de la culotte en utilisation.
